(19) Europäisches Patentamt
European Patent Office
Office européen des brevets

(11) EP 0 545 318 B1

(12) **EUROPEAN PATENT SPECIFICATION**

(45) Date of publication and mention of the grant of the patent:
**14.01.1998 Bulletin 1998/03**

(51) Int Cl.6: **C07D 331/02**

(21) Application number: **92120319.6**

(22) Date of filing: **27.11.1992**

# (54) Process for preparing alkylene sulfides

Verfahren zur Herstellung von Alkylensulfide

Procédé pour la préparation de sulfures d'alkylènes

(84) Designated Contracting States:
**BE DE FR GB NL**

(30) Priority: **29.11.1991 JP 316657/91**
**29.11.1991 JP 316658/91**
**26.11.1992 JP 317423/92**

(43) Date of publication of application:
**09.06.1993 Bulletin 1993/23**

(73) Proprietor: **NIPPON SHOKUBAI CO., LTD.**
**Chuo-ku, Osaka-shi, Osaka-fu 541 (JP)**

(72) Inventors:
- **Yano, Hitoshi**
  **Osaka 564 (JP)**
- **Yamaguchi, Yoshinari**
  **Osaka 564 (JP)**
- **Ariyoshi, Kimio**
  **Osaka 564 (JP)**
- **Shimasaki, Yuuji**
  **Osaka 564 (JP)**
- **Ishikawa, Ryuichi**
  **Osaka 593 (JP)**
- **Ueshima, Michio**
  **Takarazuka-shi, Hyogo 665 (JP)**

(74) Representative: **Casalonga, Axel**
**BUREAU D.A. CASALONGA - JOSSE**
**Morassistrasse 8**
**80469 München (DE)**

(56) References cited:
**NL-A- 7 001 172**

Remarks:
The file contains technical information submitted after the application was filed and not included in this specification

EP 0 545 318 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

BACKGROUND OF THE INVENTION

1. Field of the Invention

The present invention relates to a process for preparing alkylene sulfides by the intramolecular dehydration reaction of mercaptoalkanols in a gaseous phase under the presence of catalysts.

Alkylene sulfides, which have superior reactivity, are useful compounds that are extensively used as manufacturing materials for pharmaceuticals, pesticides, and various industrial chemicals as well as being used as a reactant for sulfur-containing polymers.

2. Description of the Prior Art

Concerning processes for manufacturing alkylene sulfides, U.S. Pat. No. 3687976 as well as U.K. Pat. No. 1135800 discloses a process wherein ethylene oxide reacts with carbonyl sulfide or carbon disulfide under the presence of catalysts. However, this process has problems of the toxicity of carbonyl sulfide and carbon disulfide as well as of the low yield of alkylene sulfide as a target product.

Further, U.S. Pat. No. 3622597 and J. Chem. Soc. (C), p1252-1256, 1969, disclose a process for preparing alkylene sulfides, wherein a mercaptoethanol is subjected to the intramolecular dehydration reaction in a liquid phase under the presence of a catalyst of sulfuric acid series. However, this process suffers from drawbacks wherein the yield of alkylene sulfide as a target product is low because of a large amount of polymerization by-products; and a large amount of cost is required for separating the polymerization by-products from the catalyst after the reaction since the reaction is a homogeneous reaction in the liquid phase. Therefore, it is impossible to put this process into practical industrial use.

Moreover, Netherlands Pat. No. 7001172 discloses a process for obtaining alkylene sulfides through the intramolecular dehydration reaction of mercaptoalkanols in a gaseous phase, which is carried out by using solid catalysts such as titanium oxide, zirconium oxide or niobium oxide. It is described that in accordance with this method, alkylene sulfides can be obtained in a high yield from mercaptoalkanols. However, no description is found in the specification concerning catalyst life, which is a very important factor to stably manufacture the target products, alkylene sulfides. According to examinations carried out by the inventors of the present application, in the case of using the catalysts such as titanium oxide, zirconium oxide or niobium oxide, since the acid property of the catalysts is too strong, so-called coking develops during the reaction such that deactivation of the catalysts proceeds quickly. Moreover, in addition to the quick deactivation of the catalysts, $MgTiO_3$ and $SrTiO_3$ that are disclosed in the above Laid-Open Patent Publication have extremely low activity in comparison with such a catalyst as titanium oxide. Therefore, it has been shown that this method using these catalysts fails to provide a satisfactory method from the industrial point of view.

SUMMARY OF THE INVENTION

It is an object of the present invention to provide a process by which alkylene sulfides are stably and efficiently obtained from mercaptoalkanols in a high yield for a long period.

In order to achieve the above objective, the inventors of the present application have found that, in a process for preparing alkylene sulfides from mercaptoalkanols, by making mercaptoalkanols subject to the intramolecular dehydration reaction in a gaseous phase under the presence of novel catalysts, alkylene sulfides can be stably obtained in a high yield for a long period. The catalysts employed are composite oxides having, as essential ingredients, both of at least one element selected from the group consisting of alkali metals, alkaline earth metals and thallium and at least one element selected from the group consisting of aluminum, boron, silicon and phosphorus.

More specifically, the present invention provides a process for preparing alkylene sulfides of the formula:

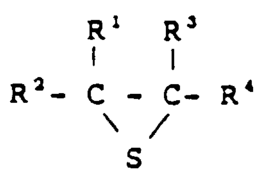

(I)

wherein $R^1$, $R^2$, $R^3$ and $R^4$ are each selected from the group consisting of a hydrogen atom, an alkyl group with 1-4 carbon atoms, a phenyl group, and a benzyl group, which is obtained through the intramolecular dehydration reaction

of mercaptoalkanols of the formula:

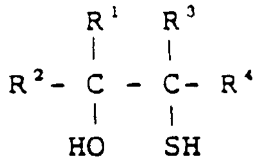 (II)

wherein $R^1$, $R^2$, $R^3$ and $R^4$ are as defined above, in a gaseous phase, the process being characterized by the use of catalysts of the formula:

$$M_aX_bY_cO_d \quad \text{(III)}$$

wherein M represents at least one element selected from the group consisting of alkali metals, alkaline earth metals and thallium; X represents at least one element selected from the group consisting of aluminum, boron, silicon and phosphorus; Y represents at least one element selected from the group consisting of lanthanides, IIIA group, IVA group, VA group, IIIB group, IVB group, VB group and VIB group (excluding an element contained in M or X); and O represents oxygen, while subscripts a, b, c and d respectively represent composition ratios of the elements; a and b are numerical values not equal to 0; b = 0.2-100 and c = 0-50 when a = 1; and d is a numerical value determined according to a, b and c.

In accordance with the present invention, alkylene sulfides are stably manufactured from mercaptoalkanols with high selectivity for a long period. Moreover, since no secondary reactants are required for the process, little by-product is produced. Thus, the present invention has succeeded in providing a very simple and economical process.

## DETAILED DESCRIPTION

Suitable mercaptoalkanols to be used as a reactant in the present invention include 2-mercaptoethanol, 1-methyl-2-mercaptoethanol, 1-ethyl-2-mercaptoethanol, 1,2-dimethyl-2-mercaptoethanol and 1-phenyl-2-mercaptoethanol. In accordance with the process of the present invention, the corresponding alkylene sulfides can be obtained depending on these mercaptoalkanols to be employed.

The catalysts to be used in the present invention are composite oxides represented by the aforementioned formula (III). These composite oxides exhibit catalytic performances in a gaseous phase reaction such that alkylene sulfides are stably obtained from mercaptoalkanols in a high yield and with a high selectivity for a long period. In particular, in the case of manufacturing ethylene sulfide which exhibits a remarkably high reactivity among alkylene sulfides, if catalysts of the formula (III) with X containing at least one element selected from the group consisting of boron and phosphorus are used, more preferable results can be obtained.

As to the reason why the catalysts of the present invention exhibit superior performances in the intramolecular dehydration reaction from mercaptoalkanols to alkylene sulfides, it has not been clarified in detail. However, active sites on the surface of the catalysts not only accelerate the intramolecular dehydration reaction of mercaptoalkanols, but also have extremely inactive characteristics toward produced alkylene sulfides; therefore, the consecutive reactions are inhibited. As a result, having less occurrence of coking, the target products can be obtained in a high yield.

Moreover, in these catalysts, special emphasis is placed on the acid and base property on the surface of the catalysts, and generally a remarkable effect has been found especially in the life of the catalysts which have acid sites ranging from +3.3 to +7.0 and base sites ranging from +7.0 to +9.3 in the acid-base strength Ho that is measured by the Hammett's indicator method.

Additionally, the acid-base strength Ho is measured through the following method.

O.1 g of a catalyst, which has been dried at 250 °C for two hours, was immersed into 1 ml of anhydrous benzene in a test tube, and ten of these samples were prepared. Then, two or three drops of benzene solutions containing the following indicators were added to the respective samples, and stirred, and then left at rest at room temperature for 24 hours. The benzene solutions used as Hammett's indicators were: dicinnamal acetone ($H_0$ = -3.0), 4-(phenylazo) diphenylamine ($H_0$ = +1.5), p-dimethylaminoazobenzene ($H_0$ = +3.3), phenylazonaphthylamine ($H_0$ = +4.0), methyl red ($H_0$ = +4.8), neutral red ($H_0$ = +6.8), bromthymol blue ($H_0$ = +7.2), m-nitrophenol ($H_0$ = +8.3), phenolphthalein ($H_0$ = +9.3), and 2,4,6-trinitroaniline ($H_0$ = +12.2). The ($H_0$) value of the acid site of the sample was determined by the smallest ($H_0$) value among those of the Hammett's indicators which had been subjected to color changes and which had the ($H_0$) value of less than +7.0. Similarly, the ($H_0$) value of the base site of the sample was determined by the greatest

($H_0$) value among those of the Hammett's indicators which had been subjected to color changes and which had the ($H_0$) value of more than +7.0.

As for starting materials for preparation of the catalysts, various materials such as metal elements, oxides, hydroxides, halides, nitrates, sulfates, phosphates, and carbonates may be employed.

The preparation method of the catalysts is not necessarily limited to a specific one; various preparation methods that are commonly used may be adopted. For example, those methods include: a method wherein various catalyst materials, which are dissolved or suspended in water and heated and concentrated while stirring, are formed after being dried and further calcined to form a catalyst; a method wherein various catalyst materials, which are dissolved or suspended in water and changed into a hydroxide by adding ammonia water thereto, are filtrated and rinsed in water, and then dried to form a catalyst; and a method wherein oxides or hydroxides of various elements are ground and mixed, and after being dried and formed, are calcined to form a catalyst. As for the calcining temperatures of the catalysts, although they depend on the materials to be used and on the calcining time, temperatures of 300°C - 1000°C are suitable in the air or in an atmosphere of inert gas (such as nitrogen, argon, or helium). Further, the catalysts of the present invention may be used in such a manner wherein they are carried on inert supports, for example, such as diatomaceous earth, kaolinite, silicon carbide, or silicon nitride.

In carrying out the gaseous-phase reaction of the present invention, any type of reactor may be employed among the fixed bed flowing type, the fluidized bed type and the moving bed type.

Moreover, mercaptoalkanol as reactant may be supplied into the reactor, either diluted or not diluted. In the case of supplying in a diluted state, an inactive gas such as nitrogen, helium or argon may be used as a diluent gas. Here, the concentration of the reactant is preferably set to not less than 5% by volume since an extremely low concentration of the reactant causes a lowering in the productivity. Generally, the reaction is conducted under a reduced pressure or a normal pressure; yet, the reaction may be conducted under a pressurized atmosphere.

The space velocity of the reactant gas, although it varies depending on the composition of the reactant gas, the reaction temperature and the reaction pressure, is set in the range of 10 - 10000 $h^{-1}$ during the reaction. The reaction temperature is in the range of 180°C - 350°C, preferably 200°C - 320°C. If the reaction temperature is below 180°C, the catalyst activity will be too low to get a sufficient yield. On the contrary, if the reaction temperature is above 350°C, secondary reactions will occur preferentially so that the selectivity of the target alkylene sulfides becomes low.

The following description will discuss the present invention in detail by reference to experimental examples and comparative examples. Here, the conversion of mercaptoalkanols, the selectivity of alkylene sulfides and the yield are determined according to the following definitions.

The conversion of mercaptoalkanols (%) = (the number of moles of mercaptoalkanols consumed through the reaction/the number of moles of mercaptoalkanols supplied) × 100.

The selectivity of alkylene sulfides (%) = (the number of moles of alkylene sulfides produced/the number of moles of mercaptoalkanols consumed) × 100.

The yield of alkylene sulfides (%) = (the number of moles of alkylene sulfides produced/the number of moles of mercaptoalkanols supplied) × 100.

## EXAMPLE 1

To a solution prepared by dissolution of 1.2 grams of cesium nitrate and 3.9 grams of boric acid in 250 ml of water was added 34 grams of silica gel. The mixture was heated and concentrated, and then dried at 130°C for 11 hours.

The resulting solid matter was calcined at 600°C in the air for 3 hours; thus, a catalyst with a composition represented by $Cs_1B_{10}Si_{90}$ in the atomic ratio excluding oxygen was obtained. The catalyst having the composition of $Cs_1B_{10}Si_{90}$ thus obtained was crushed into powder of 4-9 mesh and loaded into a reaction tube. Then, the reaction tube was immersed into a melt salt bath at 270°C, and a reactant gas was introduced into the reaction tube at a space velocity of 3000 $h^{-1}$. The reactant gas had a composition ratio of 2-mercaptoethanol:nitrogen = 10:90 in the volume ratio. The reaction conditions are shown in Tables 1 and 2.

The products of the reaction were analyzed by gas chromatography, and the results shown in Table 3 were obtained.

In the following examples 2 through 18, each catalyst was prepared by each method as shown below. Here, reactions and analyses were performed in the same manner as Example 1 except that: the kind of mercaptoalkanol, as a reactant; the concentration of a reactant gas; the space velocity; the reaction temperature; and the reaction pressure were all changed as indicated in Tables 1 and 2. Consequently, the results shown in Tables 3 were obtained.

EXAMPLE 2

A solution was prepared by dissolution of 4.2 grams of sodium nitrate and 367.8 grams of aluminum nitrate in 2000 ml of water. Then, a 28% solution of ammonia water was added to the solution with stirring carried out for two hours while maintaining the pH of the solution in the range from 8 to 10. After cooling-off, filtration, and water rinse processes, white powder was obtained. The powder was dried for 14 hours at 120°C, and then calcined at 700°C in the air for three hours; consequently, a catalyst with a composition represented by $Na_1Al_{20}$ in the atomic ratio excluding oxygen was obtained.

EXAMPLE 3

To a solution prepared by dissolution of 7.6 grams of rubidium nitrate and 0.5 grams of sodium nitrate in 170 ml of water was consecutively added 6.5 grams of a 85% solution of phosphoric acid and 34 grams of silica. The mixture was heated and concentrated, and then dried at 140°C for 12 hours.

The resulting solid matter was calcined at 600°C in the air for three hours; consequently, a catalyst with a composition represented by $Rb_{0.9}Na_{0.1}P_1Si_{10}$ in the atomic ratio excluding oxygen was obtained.

EXAMPLES 4 AND 5

4.2 grams of sodium hydroxide (purity 95%), 8.1 grams of a 85% solution of orthophosphoric acid, and 30 grams of silicon carbide (whisker) as a support were suspended in 200 ml of water, and the mixture was heated and concentrated at 80°C with sufficient stirring. The resulting solid matter, after having been dried at 120°C in the air for 12 hours, was calcined at 600°C in the air for two hours; consequently, a catalyst with a composition represented by $Na_1P_{0.7}$ in the atomic ratio excluding support element and oxygen was obtained.

EXAMPLE 6

To a solution prepared by dissolution of 10.1 grams of potassium nitrate and 11.9 grams of diammonium hydrogen phosphate in 200 ml of water was added 30 grams of silica gel. The mixture was heated and concentrated, and then dried at 120°C for 12 hours. The resulting solid matter was calcined at 600°C in the air for three hours; consequently, a catalyst with a composition represented by $K_1P_{0.9}Si_5$ in the atomic ratio excluding oxygen was obtained.

EXAMPLE 7

To a solution prepared by dissolution of 8.2 grams of boric acid and 2.1 grams of thallium nitrate in 200 ml of hot water having a temperature of 80°C were added 5.9 grams of barium hydroxide octahydrate and 70.8 grams of niobium pentoxide in powder. The mixture was heated and concentrated, and then sufficiently dried at 120°C. The resulting solid matter was calcined at 600°C in the air for two hours; consequently, a catalyst with a composition represented by $Tl_{0.3}Ba_{0.7}B_5Nb_{20}$ in the atomic ratio excluding oxygen was obtained.

EXAMPLE 8

A solution prepared by dissolution of 6.2 grams of potassium hydroxide (purity 90%) and 30.9 grams of boric acid in 300 ml of hot water having a temperature of 80°C was added to 56.5 grams of yttrium oxide in powder. The mixture was heated and concentrated, and then sufficiently dried at 120°C in the air. The resulting solid matter was calcined at 650°C in the air for six hours; consequently, a catalyst with a composition represented by $K_1B_5Y_5$ in the atomic ratio

excluding oxygen was obtained.

EXAMPLE 9

A mixture was prepared by mixing 2.9 grams of cerium(IV) oxide and 40 grams of titanium oxide, both in powder. To the mixture was added a mixed solution prepared by dissolution of 16.9 grams of potassium nitrate and 22.0 grams of diammonium hydrogen phosphate in 300 ml of water. The mixture was heated and concentrated, and then sufficiently dried at 120°C. The resulting solid matter was calcined at 600°C in the air for two hours; consequently, a catalyst with a composition represented by $K_1P_1Ce_{0.1}Ti_3$ in the atomic ratio excluding oxygen was obtained.

EXAMPLE 10

A solution was prepared by dissolution of 86.6 grams of lanthanum nitrate hexahydrate and 3.9 grams of cesium nitrate in 500 ml of water. To the solution was added 26.5 grams of diammonium hydrogen phosphate, and the mixture was heated and concentrated with sufficient stirring, and then sufficiently dried at 120°C. The resulting solid matter was calcined at 750°C in the air for three hours; consequently, a catalyst with a composition represented by $Cs_1P_{10}La_{10}$ in the atomic ratio excluding oxygen was obtained.

EXAMPLE 11

To a solution prepared by dissolution of 5.3 grams of strontium nitrate and 2.1 grams of sodium nitrate in 300 ml of water was added 61.6 grams of zirconium oxide in powder with sufficient stirring. Then, 4.0 grams of diammonium hydrogen phosphate was added to the mixture, and it was heated and concentrated, and then sufficiently dried at 130°C. The resulting powder was calcined at 650°C in the air for three hours; consequently, a catalyst whose composition was represented by $Na_{0.5}Sr_{0.5}P_{0.6}Zr_{10}$ in the atomic ratio excluding oxygen was obtained.

EXAMPLE 12

A mixture prepared by mixing and grinding 16.5 grams of sodium tungstate dihydrate and 30 grams of silica gel in a mortar was further kneaded with 100 ml of water. The resulting slurry was sufficiently dried at 130°C, and then calcined at 600°C in the air for three hours; consequently, a catalyst with a composition represented by $Na_1Si_5W_{0.5}$ in the atomic ratio excluding oxygen was obtained.

EXAMPLE 13

To a solution prepared by dissolution of 88.0 grams of diammonium hydrogen phosphate in 800 ml of water was added 74.1 grams of calcium hydroxide, and the mixture was evaporated and dried hard, thereby resulting in a solid matter. The solid matter was calcined at 600 °C in the air for three hours; consequently, a catalyst with a composition represented by $Ca_1P_{0.66}$ in the atomic ratio excluding oxygen was obtained.

EXAMPLE 14

To a solution prepared by dissolution of 321.7 grams of lithium nitrate and 205.3 grams of diammonium hydrogen phosphate in 300 ml of water was added a 28% ammonia water so as to adjust the pH of the solution to 8 - 10 with stirring carried out for two hours. Then, the mixture was cooled off, filtered and rinsed in water, thereby resulting in white powder. The white powder was calcined at 600°C in the air for three hours; consequently, a catalyst with a composition represented by $Li_1P_{0.33}$ in the atomic ratio excluding oxygen was obtained.

EXAMPLE 15

To a solution prepared by dissolution of 43.4 grams of strontium nitrate in 200 ml of water was added 50 grams of aluminum phosphate. The solution was heated and concentrated, and then dried at 130°C for ten hours. The resulting solid matter was calcined at 600°C in the air for three hours; consequently, a catalyst with a composition represented by $Sr_1Al_2P_2$ in the atomic ratio excluding oxygen was obtained.

EXAMPLE 16

A mixture was prepared by mixing and grinding in a mortar 3.5 grams of gallium oxide in powder and 50 grams of

kaolin powder as a support. Then, to the mixture was added a solution prepared by dissolution of 9 grams of sodium dihydrogen phosphate in 300 ml of water, and the mixture was heated and concentrated. The resulting solid matter was sufficiently dried at 120°C, and then calcined at 500°C in the air for six hours; consequently, a catalyst with a composition represented by $Na_1P_1Ga_{0.5}$ in the atomic ratio excluding support atoms and oxygen was obtained.

EXAMPLE 17

A mixture was prepared by sufficiently mixing and grinding in a mortar 5.4 grams of boric acid, 3.9 grams of barium hydroxide octahydrate, 40 grams of titanium oxide in powder, and 13.1 grams of germanium oxide in powder. After adding 200 ml of 50°C warm water thereto, the mixture was heated and concentrated. The resulting solid matter was sufficiently dried at 120°C, and then calcined at 750°C in the air for three hours; consequently, a catalyst with a composition represented by $Ba_1B_7Ge_{10}Ti_{40}$ in the atomic ratio excluding oxygen was obtained.

EXAMPLE 18

A solution was prepared by dissolution of 10.5 grams of dipotassium hydrogen phosphate in 200 ml of water. After adding thereto 1.7 grams of antimony(III) oxide in powder and 30.6 grams of alumina powder, the mixture was heated and concentrated. The resulting solid matter was sufficiently dried at 120°C and then calcined at 800°C in the air for four hours; consequently, a catalyst with a composition represented by $K_1P_{0.5}Al_5Sb_{0.2}$ in the atomic ratio excluding oxygen was obtained.

Table 1

| Example | Catalyst Composition | | | Acid-Base Strength $H_0$ | |
|---|---|---|---|---|---|
| | $M_a$ | $X_b$ | $Y_c$ | Acid Site | Base Site |
| 1 | $Cs_1$ | $B_{10}Si_{90}$ | - | +3.3 | +7.2 |
| 2 | $Na_1$ | $Al_{20}$ | - | +4.0 | +7.2 |
| 3 | $Na_{0.1}Rb_{0.9}$ | $P_1Si_{10}$ | - | +4.0 | +8.3 |
| 4 | $Na_1$ | $P_{0.7}$ | - | +6.8 | +8.3 |
| 5 | $Na_1$ | $P_{0.7}$ | - | +6.8 | +8.3 |
| 6 | $K_1$ | $P_{0.9}Si_5$ | - | +4.8 | +8.3 |
| 7 | $Tl_{0.3}Ba_{0.7}$ | $B_5$ | $Nb_{20}$ | +3.3 | +7.2 |
| 8 | $K_1$ | $B_5$ | $Y_5$ | +4.0 | +7.2 |
| 9 | $K_1$ | $P_1$ | $Ce_{0.1}Ti_3$ | +4.0 | +7.2 |
| 10 | $Cs_1$ | $P_{10}$ | $La_{10}$ | +4.8 | +8.3 |
| 11 | $Na_{0.5}Sr_{0.5}$ | $P_{0.6}$ | $Zr_{10}$ | +4.0 | +8.3 |
| 12 | $Na_1$ | $Si_5$ | $W_{0.5}$ | +4.8 | +8.3 |
| 13 | $Ca_1$ | $P_{0.66}$ | - | +6.8 | +9.3 |
| 14 | $Li_1$ | $P_{0.33}$ | - | +4.8 | +9.3 |
| 15 | $Sr_1$ | $Al_2P_2$ | - | +6.8 | +8.3 |
| 16 | $Na_1$ | $P_1$ | $Ga_{0.5}$ | +4.0 | +8.3 |
| 17 | $Ba_1$ | $B_7$ | $Ge_{10}Ti_{40}$ | +4.8 | +8.3 |
| 18 | $K_1$ | $P_{0.5}Al_5$ | $Sb_{0.2}$ | +6.8 | +9.3 |

Table 2

| Example | Reactant | Space Velocity ($h^{-1}$) | Temp. (°C) | Reactant Ratio (Vol. %) | Pressure (mmHg) |
|---|---|---|---|---|---|
| 1 | 2-mercaptoethanol | 3000 | 270 | 10 | 760 |

Table 2 (continued)

| Example | Reactant | Space Velocity ($h^{-1}$) | Temp. (°C) | Reactant Ratio (Vol. %) | Pressure (mmHg) |
|---|---|---|---|---|---|
| 2 | 1,2-dimethyl-2-mercaptoethanol | 2000 | 250 | 20 | 760 |
| 3 | 2-mercaptoethanol | 4000 | 270 | 10 | 760 |
| 4 | 2-mercaptoethanol | 3000 | 270 | 10 | 760 |
| 5 | 2-mercaptoethanol | 400 | 300 | 100 | 80 |
| 6 | 2-mercaptoethanol | 2000 | 250 | 10 | 760 |
| 7 | 1-methyl-2-mercaptoethanol | 2000 | 270 | 10 | 760 |
| 8 | 2-mercaptoethanol | 4000 | 300 | 20 | 760 |
| 9 | 2-mercaptoethanol | 2500 | 300 | 20 | 760 |
| 10 | 2-mercaptoethanol | 2000 | 230 | 10 | 760 |
| 11 | 1-methyl-2-mercaptoethanol | 200 | 200 | 100 | 150 |
| 12 | 1-methyl-2-mercaptoethanol | 1500 | 300 | 10 | 760 |
| 13 | 2-mercaptoethanol | 300 | 300 | 100 | 80 |
| 14 | 1-phenyl-2-mercaptoethanol | 7000 | 300 | 5 | 1000 |
| 15 | 1,2-dimethyl-2-mercaptoethanol | 200 | 270 | 100 | 80 |
| 16 | 2-mercaptoethanol | 2000 | 300 | 10 | 760 |
| 17 | 2-mercaptoethanol | 2500 | 270 | 10 | 760 |
| 18 | 2-mercaptoethanol | 2000 | 300 | 10 | 760 |

Table 3

| Example | Product | Elapsed Time (hr) | Conversion (%) | Selectivity (%) | Yield (%) |
|---|---|---|---|---|---|
| 1 | ethylene sulfide | 1<br>200 | 87.4<br>84.0 | 95.1<br>95.1 | 83.1<br>79.9 |
| 2 | 1,2-dimethyl ethylene sulfide | 1<br>50 | 87.2<br>84.9 | 93.1<br>95.6 | 81.2<br>81.1 |
| 3 | ethylene sulfide | 1<br>100 | 86.3<br>86.2 | 95.4<br>96.1 | 82.3<br>82.8 |
| 4 | ethylene sulfide | 2<br>200 | 95.1<br>93.7 | 93.2<br>93.4 | 88.6<br>87.5 |
| 5 | ethylene sulfide | 2<br>200 | 93.6<br>90.8 | 97.1<br>97.5 | 90.9<br>88.5 |
| 6 | ethylene sulfide | 2<br>200 | 97.1<br>96.5 | 98.3<br>98.7 | 95.4<br>95.2 |
| 7 | 2-methyl ethylene sulfide | 1<br>100 | 84.4<br>83.0 | 92.2<br>91.9 | 77.8<br>76.3 |

Table 3 (continued)

| Example | Product | Elapsed Time (hr) | Conversion (%) | Selectivity (%) | Yield (%) |
|---|---|---|---|---|---|
| 8 | ethylene sulfide | 2<br>100 | 93.6<br>91.5 | 94.7<br>95.3 | 88.6<br>87.2 |
| 9 | ethylene sulfide | 2<br>200 | 93.8<br>93.5 | 92.6<br>92.8 | 86.9<br>86.8 |
| 10 | ethylene sulfide | 2<br>100 | 87.2<br>82.9 | 93.3<br>92.5 | 81.4<br>76.7 |
| 11 | 2-methyl ethylene sulfide | 2<br>100 | 95.9<br>95.5 | 96.2<br>95.8 | 92.3<br>91.5 |
| 12 | 2-methyl ethylene sulfide | 2<br>50 | 88.8<br>85.9 | 91.6<br>92.3 | 81.3<br>79.3 |
| 13 | ethylene sulfide | 1<br>100 | 90.6<br>90.0 | 94.6<br>94.3 | 85.7<br>84.9 |
| 14 | 2-phenyl ethylene sulfide | 1<br>100 | 84.8<br>83.2 | 97.7<br>97.9 | 82.8<br>81.5 |
| 15 | 1,2-dimethyl ethylene sulfide | 1<br>100 | 89.4<br>87.1 | 91.6<br>93.3 | 81.9<br>81.3 |
| 16 | ethylene sulfide | 1<br>100 | 92.3<br>89.9 | 91.1<br>93.7 | 84.1<br>84.2 |
| 17 | ethylene sulfide | 1<br>100 | 87.6<br>85.9 | 94.1<br>93.7 | 82.4<br>80.5 |
| 18 | ethylene sulfide | 1<br>100 | 96.3<br>93.0 | 92.4<br>91.9 | 89.0<br>85.5 |

COMPARATIVE EXAMPLES 1-4

Concerning each of the catalysts prepared in accordance with the following comparative examples 1-4, reactions and analyses were performed in the same manner as Example 1 except that: the kind of mercaptoalkanol, as a reactant; the concentration of a reactant gas; the space velocity; the reaction temperature; and the reaction pressure were changed as indicated in Table 4. Consequently, the results shown in Table 5 were obtained.

COMPARATIVE EXAMPLE 1

A mixture prepared by addition of 50 grams of commercial niobium pentoxide to 100 ml of water was heated and concentrated, and then sufficiently dried at 150°C. The resulting solid matter was calcined at 600°C in the air for three hours; consequently, a catalyst with a composition represented by $Nb_1$ in the atomic ratio excluding oxygen was obtained.

COMPARATIVE EXAMPLE 2

A mixture prepared by addition of 50 grams of titanium oxide (rutile type) in powder to 100 ml of water was heated and concentrated, and then dried at 150°C. The resulting solid matter was calcined at 600°C in the air for three hours; consequently, a catalyst with a composition represented by $Ti_1$ in the atomic ratio excluding oxygen was obtained.

COMPARATIVE EXAMPLE 3

A mixture prepared by addition of 50 grams of commercial strontium titanate to 100 ml of water was heated and concentrated, and then dried at 150°C. The resulting solid matter was calcined at 500°C in the air for three hours;

consequently, a catalyst with a composition represented by $Sr_1Ti_1$ in the atomic ratio excluding oxygen was obtained.

COMPARATIVE EXAMPLE 4

To a solution prepared by dissolution of 8.8 grams of diammonium hydrogen phosphate in 130 ml of water was added 40 grams of silica gel. The mixture was heated and concentrated, and then dried at 140°C for ten hours. The resulting solid matter was calcined at 600°C in the air for three hours; consequently, a catalyst with a composition represented by $P_1Si_{10}$ in the atomic ratio excluding oxygen was obtained.

Table 4

| * | Catalyst Composition | | | Acid-Base Stren. ($H_0$) | | Reactant | Space Velocity ($h^{-1}$) | Temp (°C) | Reactant ratio (V%) | Pressure (mmHg) |
|---|---|---|---|---|---|---|---|---|---|---|
| | $M_a$ | $X_b$ | $Y_c$ | Acid Site | Base Site | | | | | |
| 1 | - | - | $Nb_1$ | +1.5 | - | 2-mercapto ethanol | 2000 | 275 | 20 | 760 |
| 2 | - | - | $Ti_1$ | +3.3 | - | 2-mercapto ethanol | 2000 | 270 | 10 | 760 |
| 3 | $Sr_1$ | - | $Ti_1$ | +6.8 | +7.2 | 2-mercapto ethanol | 2000 | 270 | 10 | 760 |
| 4 | - | $P_1Si_{10}$ | - | -3.0 | - | 2-mercapto ethanol | 2000 | 250 | 20 | 760 |

* Comparative Example

Table 5

| Comparative Example | Product | Elapsed Time (hr) | Conversion (%) | Selectivity (%) | Yield (%) |
|---|---|---|---|---|---|
| 1 | ethylene sulfide | 1<br>30 | 95.1<br>48.5 | 70.0<br>67.8 | 66.6<br>32.9 |
| 2 | ethylene sulfide | 1<br>30 | 86.7<br>36.4 | 71.1<br>73.9 | 61.6<br>26.8 |
| 3 | ethylene sulfide | 1<br>30 | 16.2<br>10.8 | 76.3<br>73.9 | 12.4<br>8.0 |
| 4 | ethylene sulfide | 1 | 93.4 | 3.2 | 3.0 |

**Claims**

**1.** A process for preparing alkylene sulfides of the formula

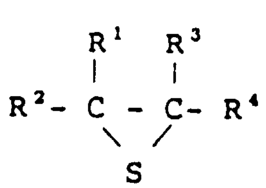

(I)

wherein $R^1$, $R^2$, $R^3$ and $R^4$ are each selected from the group consisting of a hydrogen atom, an alkyl group with 1-4 carbon atoms, a phenyl group, and a benzyl group, comprising:

carrying out the intramolecular dehydration reaction of mercaptoalkanols of the formula

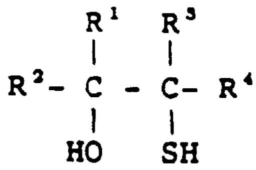

(II)

wherein $R^1$, $R^2$, $R^3$ and $R^4$ are as defined above, in a gaseous phase at a reaction temperature in the range from 180°C to 350°C, in the presence of catalysts of the formula

$$M_aX_bY_cO_d \quad \text{(III)}$$

wherein M represents at least one element selected from the group consisting of alkali metals, alkaline earth metals and thallium; X represents at least one element selected from the group consisting of aluminum, boron, silicon and phosphorus; Y represents at least one element selected from the group consisting of lanthanides, IIIA group, IVA group, VA group, IIIB group, IVB group, VB group and VIB group, excluding an element contained in M or X; O represents oxygen; and subscripts a, b, c and d respectively represent composition ratios of the elements; a and b are numerical values not equal to 0; b = 0.2-100 and c = 0-50 when a = 1; and d is a numerical value which is consistent with the values of a, b and c so as to give a neutrally charged catalyst moiety.

**2.** The process as defined in claim 1, wherein in formula (III), c is a value not equal to 0; and Y represents at least one element selected from the group consisting of lanthanum, cerium, yttrium, titanium, zirconium and niobium.

**3.** The process as defined in claim 1, wherein in formula (III), c is equal to 0.

**4.** The process as defined in claim 1, wherein in formula (III), X includes at least one element selected from the group consisting of boron and phosphorus.

**5.** The process as defined in claim 1, wherein the catalyst has acid sites ranging from +3.3 to +7.0 and base sites ranging from +7.0 to +9.3 in the acid-base strength $H_0$ that is measured by the Hammett's indicator method.

**6.** The process for preparing alkylene sulfides as defined in claim 1, wherein alkylene sulfide and mercaptoalkanol are ethylene sulfide and 2-mercaptoethanol respectively.

**7.** The process for preparing alkylene sulfides as defined in claim 6, wherein in formula (III), X includes at least one element selected from the group consisting of boron and phosphorus.

**8.** The process for preparing alkylene sulfides as defined in claim 1, wherein the reaction is carried out at a temperature in the range from 200°C to 320°C.

**9.** The process for preparing alkylene sulfides as defined in claim 1, wherein the concentration of the mercaptoalkanol is not less than 5% by volume.

**Patentansprüche**

**1.** Verfahren zur Herstellung von Alkylensulfiden der Formel:

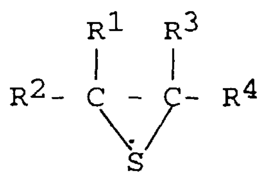

(I)

worin $R^1$, $R^2$, $R^3$ und $R^4$ jeweils ausgewählt sind aus einem Wasserstoffatom, einer Alkyl-Gruppe mit 1 bis 4 Kohlenstoffatomen, einer Phenyl-Gruppe und einer Benzyl-Gruppe, umfassend die Durchführung der intramolekularen Dehydratisierungsreaktion von Mercaptoalkanolen der Formel:

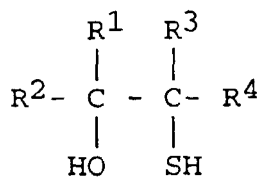

(II)

worin $R^1$, $R^2$, $R^3$ und $R^4$ wie oben definiert sind, in der Gasphase, bei einer Reaktionstemperatur im Bereich von 180 bis 350°C, in Gegenwart von Katalysatoren der Formel:

$$M_aX_bY_cO_d \qquad \text{(III)}$$

worin M mindestens ein Element, ausgewählt aus Alkalimetallen, Erdalkalimetallen und Thallium repräsentiert, X repräsentiert mindestens ein Element, ausgewählt aus Aluminium, Bor, Silicium und Phosphor, Y repräsentiert mindestens ein Element, ausgewählt aus den Lanthaniden, der Gruppe IIIA, der Gruppe IVA, der Gruppe VA, der Gruppe IIIB, der Gruppe IVB, der Gruppe VB und der Gruppe VIB, wobei in M oder X enthaltene Elemente ausgeschlossen sind, O repräsentiert Sauerstoff, und die Indices a, b, c und d repräsentieren jeweils die Zusammensetzungsverhältnisse der Elemente; a und b sind numerische Werte ungleich 0, b = 0,2 - 100 und c = 0 - 50 wenn a = 1 ist; und d ist ein numerischer Wert, der mit den Werten von a, b und c konsistent ist, so daß eine neutral geladene Katalysatoreinheit erhalten wird.

**2.** Verfahren gemäß Anspruch 1, worin c in Formel (III) ein Wert ungleich 0 ist, und Y repräsentiert mindestens ein Element, ausgewählt aus Lanthan, Cer, Yttrium, Titan, Zirkon und Niob.

**3.** Verfahren gemäß Anspruch 1, worin c in Formel (III) 0 ist.

**4.** Verfahren gemäß Anspruch 1, worin X in Formel (III) mindestens ein Element, ausgewählt aus Bor und Phosphor, einschließt.

**5.** Verfahren gemäß Anspruch 1, worin der Katalysator saure Orte im Bereich von +3,3 bis +7,0 und Basenorte im Bereich von +7,0 bis +9,3 in der Säure-Basen-Stärke $H_0$, gemessen nach der Hammett-Indikatormethode, aufweist.

**6.** Verfahren zur Herstellung von Alkylensulfiden gemäß Anspruch 1, worin das Alkylensulfid und das Mercaptoalkanol Ethylensulfid bzw. 2-Mercaptoethanol ist.

**7.** Verfahren zur Herstellung von Alkylensulfiden gemäß Anspruch 6, worin X in Formel (III) mindestens ein Element, ausgewählt aus Bor und Phosphor, einschließt.

**8.** Verfahren zur Herstellung von Alkylensulfiden gemäß Anspruch 1, worin die Reaktion bei einer Temperatur von 200 bis 320°C durchgeführt wird.

**9.** Verfahren zur Herstellung von Alkylensulfiden gemäß Anspruch 1, worin die Konzentration an Mercaptoalkanol

nicht weniger als 5 Vol.-% beträgt.

**Revendications**

1. Procédé de préparation de sulfures d'alkylène de formule :

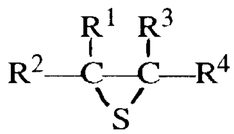 (I)

dans laquelle $R^1$, $R^2$, $R^3$ et $R^4$ représentent chacun un membre de l'ensemble constitué par l'atome d'hydrogène, les groupes alkyle comportant de 1 à 4 atomes de carbone, le groupe phényle et le groupe benzyle, lequel procédé comporte le fait d'effectuer une réaction de déshydratation intramoléculaire de mercaptoalcanols de formule :

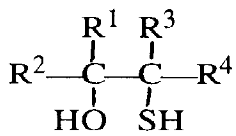 (II)

dans laquelle les symboles $R^1$, $R^2$, $R^3$ et $R^4$ ont les mêmes significations que ci-dessus,
en phase gazeuse, à une température située dans l'intervalle allant de 180 °C à 350 °C, et en présence de catalyseurs de formule

$$M_aX_bY_cO_d \qquad (III)$$

dans laquelle M représente au moins un élément choisi dans l'ensemble que constituent les métaux alcalins, les métaux alcalino-terreux et le thallium, X représente au moins un élément choisi dans l'ensemble que constituent l'aluminium, le bore, le silicium et le phosphore, Y représente au moins un élément choisi dans l'ensemble que constituent la série des lanthanides et les groupes IIIA, IVA, VA, IIIB, IVB, VB et VIB (à l'exclusion des éléments déjà représentés par M ou X), et O représente l'oxygène, et dans laquelle les indices a, b, c et d représentent les rapports dans lesquels les divers éléments apparaissent dans la composition, a et b sont des nombres non nuls, b vaut de 0,2 à 100 et c vaut de 0 à 50 si a vaut 1, et d est un nombre dont la valeur s'accorde aux valeurs de a, b et c de façon que le catalyseur soit électriquement neutre.

2. Procédé conforme à la revendication 1, dans lequel, dans la formule (III), c est un nombre non nul et Y représente au moins un élément choisi dans l'ensemble que constituent le lanthane, le cérium, l'yttrium, le titane, le zirconium et le niobium.

3. Procédé conforme à la revendication 1, dans lequel, dans la formule (III), c vaut 0.

4. Procédé conforme à la revendication 1, dans lequel, dans la formule (III), X englobe au moins un élément choisi parmi le bore et le phosphore.

5. Procédé conforme à la revendication 1, dans lequel le catalyseur possède des sites acides et des sites basiques dont la force acide-base $H_0$, mesurée selon la méthode des indicateurs de Hammet, vaut respectivement de +3,3 à +7,0 et de +7,0 à +9,3.

6. Procédé de préparation de sulfures d'alkylène, conforme à la revendication 1, dans lequel le sulfure d'alkylène et le mercaptoalcanol sont respectivement du sulfure d'éthylène et du 2-mercaptoéthanol.

7. Procédé de préparation de sulfures d'alkylène, conforme à la revendication 6, dans lequel, dans la formule (III), X englobe au moins un élément choisi parmi le bore et le phosphore.

8. Procédé de préparation de sulfures d'alkylène, conforme à la revendication 1, dans lequel on effectue la réaction à une température située dans l'intervalle allant de 200 °C à 320 °C.

9. Procédé de préparation de sulfures d'alkylène, conforme à la revendication 1, dans lequel la concentration du mercaptoalcanol vaut au moins 5 % en volume.